# EUROPEAN PATENT APPLICATION

(11) **EP 4 659 744 A1**
(43) Date of publication of application: **10.12.2025**
(21) Application number: 25178871.7
(22) Date of filing: 26.05.2025
(51) Int. Cl.: A61K 9/20, A61K 31/44

(54) **THE PROLONGED-RELEASE TABLET COMPRISING FAMPRIDINE**

(30) Priority: 28.05.2024 TR 2024066607
(71) Applicant: Sanovel Ilac Sanayi Ve Ticaret Anonim Sirketi, Istanbul (TR)
(72) Inventor: SUNEL, Fatih, Istanbul (TR); ATAK, Fadime Bilgehan, Istanbul (TR); IPEK, Celaleddin, Istanbul (TR); PEHLIVAN AKALIN, Nur, Istanbul (TR); ULUSOY BOZYEL, Muge, Istanbul (TR); YENI, Songul, Istanbul (TR); DAGCIOGLU, Erdal, Istanbul (TR)
(74) Representative: Genç Ilhan, Oznur

(57) **Abstract**

The present invention relates to a prolonged-release tablet comprising fampridine and at least one pharmaceutically acceptable excipient wherein a d (0.9) particle size of fampridine is smaller than 120 µm and at least one cellulosic polymer for prolonged-release.

## Description

### Field of the invention

The present invention relates to a prolonged-release tablet comprising fampridine and at least one pharmaceutically acceptable excipient wherein a d (0.9) particle size of fampridine is smaller than 120 µm and at least one cellulosic polymer for prolonged-release.

### Background of the invention

Multiple Sclerosis (MS) is a chronic disease of the central nervous system (CNS) resulting in loss of muscle control, balance, and sensation. Autoimmunity, infectious agents, environmental triggers and hereditary factors influential in disease development.

Myelin provides a covering or insulation for nerves, improves the conduction of impulses along the nerves and also is important for maintaining the health of the nerves. In multiple sclerosis, inflammation causes the myelin to disappear. So, the electrical impulses become slower. In addition, the nerves themselves are damaged. Patient suffers from a range of symptoms which affect their health-related quality of life such as pain, muscle spasticity and spasm, bladder problems and sleep disturbance.

Multiple sclerosis (MS) is the most common autoimmune disorder affecting the central nervous system. Multiple sclerosis, also known as disseminated sclerosis or encephalomyelitis disseminate, is a demyelinating disease in which the insulating covers of nerve cells in the brain and spinal cord are damaged. This damage disrupts the ability of parts of the nervous system to communicate, resulting in a wide range of signs and symptoms, including physical, mental and sometimes psychiatric problems.

Fampridine is a potassium channel blocker indicated for symptomatic improvement of walking in adults with multiple sclerosis, including relapsing remitting, secondary progressive, progressive relapsing and primary progressive. The chemical name of fampridine is 1,4-dihydropyridin-4-imine and its chemical structure is shown in Formula I.

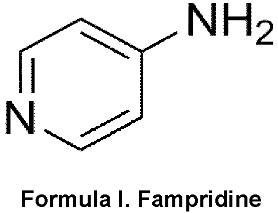

Fampridine is available under the trade name Ampyra from Acorda Therapeutics is available as 10-mg film-coated, prolonged-release, white to off-white, oval tablets.

Fampridine has been formulated in prolonged-release matrix tablets at various concentrations ranging from 5 to 40 mg.

There is no known cure for MS until now. Current treatments typically focus on accelerating patient's recovery from attacks, slowing the progression of the disease and restraining MS symptoms.

Research in patients with multiple sclerosis (MS), including phase 1, 2 and 3 clinical trials, has shown that the drug fampridine can improve many types of nerve function damaged by the disease, with particular attention paid to its effects on improving walking and leg strength.

Fampridine is known in the art for its potency in improving walking capacity in patients with multiple sclerosis. However, compositions comprising fampridine have been reported to exhibit substantial degradation upon storage. It is believed that the drug reacts with some of the excipients present in the formulation leading to increase in the level of the impurities as well as formation of unidentified impurities during storage.

WO2017058869A1 discloses sustained release fampridine tablets, which include a core and a coating. The core comprises fampridine, a polyethylene oxide with a molecular weight between about 1,000,000 and 10,000,000, and a mixture comprising polyvinyl acetate and polyvinyl pyrrolidone; and a coating comprises ethylcellulose surrounding said compressed core.

US20170071923A1 provides a sustained release oral dosage form containing fampridine that can be administered once daily. The dosage form includes fampridine as the active pharmaceutical ingredient and the excipients comprising osmotic agents in a tablet core.

In prior art, there are also several patents which disclose fampridine in oral pharmaceutical dosage forms. However, the stability and dissolution profile solutions in the prior art are difficult, costly and complex. There are no recommendations that improve both stability and dissolution profile simultaneously.

Thus, there is still a need for a prolonged-release tablet of fampridine, wherein the stability of the active pharmaceutical ingredient and content uniformity can be ensured. In present invention, a tablet in which the degradation of the active pharmaceutical ingredient is prevented or at least significantly reduced even under harsh conditions is provided. The tablet also has the desired dissolution profile.

### Detailed description of the Invention

The main object of the present invention is to a prolonged-release tablet comprising fampridine with high stability, having desired level of prolonged-release profile and content uniformity which overcomes the above-described problems of fampridine in prior art and have additive advantages over them.

Another object of the present invention is to provide a process for a prolonged-release tablet comprising fampridine. The process is a simple, rapid, cost effective, time-saving and industrially convenient method.

Targeting of drugs to the colon following oral administration has also been accomplished by using prolonged-release polymers; also they act as binders that swell when hydrated by gastric media and delay absorption. In this invention, we have used cellulose derived polymers as prolonged-release polymers.

Cellulose and its derivatives have demonstrated to be versatile materials with unique chemical structure which provides a good platform for the construction of hydrogel networks with distinctive properties as respects of swelling ability and sensibility to external stimuli. The cellulosic prolonged-release polymer provides the desired dispersion of fampridine throughout the matrix and it imparts chemical and physical stability to the composition while providing prolonged-release profile. This improved dosage stability is particularly important in compositions of the present invention containing low amounts of fampridine. By utilising a cellulosic prolonged-release polymer, stability is achieved while maintaining the desired prolonged-release profile.

Since low amounts of fampridine are used in the formulation, it is important to ensure content uniformity. When used in the particle sizes specified below, fampridine helped provide the desired dissolution profile by ensuring content uniformity.

As used here in, 'particle size' means the cumulative volume size distribution as tested by any conventionally accepted method such as the laser diffraction method (i.e. Malvern analysis). The term d (0.9) means the size at which %90 by volume of the particles are finer, the term d (0.5) means the size at which %50 by volume of the particles are finer, the term d (0.1) means the size at which %10 by volume of the particles are finer.

According to another embodiment of the present invention, a prolonged-release tablet comprises fampridine and at least one pharmaceutically acceptable excipient wherein a d (0.9) particle size of fampridine is smaller than 120 µm and at least one cellulosic prolonged-release polymer.

Suitable prolonged-release polymers are selected from the group comprising ethylcellulose, methylcellulose, hydroxypropyl cellulose, hydroxypropyl methylcellulose (HPMC), hydroxyethyl cellulose, hydroxymethyl cellulose, sodium carboxymethylcellulose, carboxymethyl cellulose, nitrocellulose, methylcellulose or mixtures thereof.

According to another embodiment of the present invention, the prolonged-release polymer is hydroxypropyl methyl cellulose. This excipient provides stability while ensuring the desired prolonged-release profile. Especially, a low-viscosity type of HPMC is used, for example; HPMC K100LV.

According to another embodiment of the present invention, the amount of hydroxypropyl methyl cellulose is between 25.0% to 70.0% by weight of the total composition. Preferably, it is between 42.0% to 65.0% by weight of the total composition. More preferably, it is between 52.0% to 65.0% by weight of the total composition.

According to one embodiment, fampridine has a d (0.9) particle size between 120 µm and 20 µm or between 100 µm and 25 µm or between 85 µm and 30 µm or between 80 µm and 35 µm. Use of particle size in this range ensures content uniformity.

According to another embodiment of the present invention, the amount of fampridine is between 0.1% to 5.0% by weight of the total composition. Preferably, it is between 1.2% to 3.5% by weight of the total composition.

In a preferred embodiment according to the present invention, the prolonged-release tablet further comprises at least one pharmaceutically acceptable excipient which is selected from fillers, lubricants, glidants, coating agents or mixtures thereof.

Suitable fillers are selected from the group comprising microcrystalline cellulose, mannitol, spray-dried mannitol, lactose, lactose monohydrate, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or mixtures thereof.

According to another embodiment of the present invention, the filler is microcrystalline cellulose.

According to another embodiment of the present invention, the amount of microcrystalline cellulose is between 25.0% to 60.0% by weight of the total composition. Preferably, it is between 30.0% to 45.0% by weight of the total composition.

Suitable lubricants are selected from the group comprising from magnesium stearate, calcium stearate, zinc stearate, talc, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyseryl palmito sulphate, sodium stearyl fumarate, sodium lauryl sulphate or mixtures thereof.

According to another embodiment of the present invention, the lubricant is magnesium stearate.

According to another embodiment of the present invention, the amount of magnesium stearate is between 0.05 to 3.0 by weight of the total composition.

Suitable glidants are selected from the group comprising anhydrous colloidal silicon dioxide, corn starch, talc or mixtures thereof.

According to another embodiment of the present invention, the glidant is anhydrous colloidal silicon dioxide.

According to another embodiment of the present invention, the amount of anhydrous colloidal silicon dioxide is between 0.05 to 3.0 by weight of the total composition. Preferably, it is between 0.1% to 1.5% by weight of the total composition.

Suitable coating agents are selected from the group comprising hydroxypropyl methyl cellulose, polymethacrylates, polyalkylacrylates copolymers, hydroxyl propyl methyl cellulose, lactose monohydrate, hydroxypropyl cellulose, polyvinyl alcohol, polyethylene glycol, talc, polyvinyl alcohol-polyethylene glycol copolymers (Kollicoat^{®} IR), ethylcellulose dispersions (Surelease^{®}), polyvinylpyrrolidone, polyvinylpyrrolidone-vinyl acetate copolymer (PVP-VA), all kinds of Opadry^{®}, pigments, dyes, titanium dioxide, iron oxide or mixtures thereof.

According to another embodiment of the present invention, the coating agent is hydroxypropyl methyl cellulose, titanium dioxide, polyethylene glycol or mixtures thereof.

According to another embodiment of the present invention, the prolonged-release tablet comprises;
- Fampridine having d (0.9) particle size smaller than 120 µm
- HPMC K100LV
- Microcrystalline cellulose PH103
- Anhydrous colloidal silicon
- Magnesium stearate

The prolonged-release tablet is prepared using direct compression, wet or dry granulation, hot melt granulation, hot melt extrusion, fluidized bed granulation, extrusion, spheronization, slugging, spray drying or solvent evaporation. In the invention, the prolonged-release tablet is obtained by direct compression.

According to one embodiment, the prolonged-release tablet has a hardness of between 180 N and 300 N. This provides high chemical and mechanical stability, thus it is not brittle easily. Hardness test is done with Erweka Tablet Hardness Tester.

### Example 1:

| **Ingredients** | **Amount (%)** | **Amount (%)** |
|---|---|---|
| Fampridine | 0.1 - 7.0 | 2.5 |
| HPMC K100LV | 25.0 - 70.0 | 60.0 |
| Microcrystalline cellulose PH103 | 25.0 - 60.0 | 36.7 |
| Anhydrous colloidal silicon | 0.05 - 3.0 | 0.4 |
| Magnesium stearate | 0.05 - 3.0 | 0.2 |
| **Total** | **100.00** | **100.00** |

a) Weighing the active substances and all excipients,
b) Mixing fampridine with HPMC K100LV geometrically,
c) Sieving the mixture through sieve.
d) Sieving anhydrous colloidal silicon and MCC PH103, then adding the mixture at step (c) and then mixing for 5 min,
e) Sieving Magnesium stearate through sieve and adding the mixture at step (d) and mixing for 3 min,
f) The prepared mixture is pressed so that the tablet weight is of the appropriate specification.
g) The tablet weight is coated until the desired film tablet weight is reached.

## Claims

1. A prolonged-release tablet comprising fampridine and at least one pharmaceutically acceptable excipient wherein a d (0.9) particle size of fampridine is smaller than 120 µm and at least one cellulosic prolonged-release polymer.

2. The prolonged-release tablet according to claim 1, wherein prolonged-release polymers are selected from the group comprising ethylcellulose, methylcellulose, hydroxypropyl cellulose, hydroxypropyl methylcellulose (HPMC), hydroxyethyl cellulose, hydroxymethyl cellulose, sodium carboxymethylcellulose, carboxymethyl cellulose, nitrocellulose, methylcellulose or mixtures thereof.

3. The prolonged-release tablet according to claim 1 or 2, wherein the prolonged-release polymer is hydroxypropyl methyl cellulose.

4. The prolonged-release tablet according to claim 3, wherein the amount of hydroxypropyl methyl cellulose is between 25.0% to 50.0% by weight of the total composition.

5. The prolonged-release tablet according to claim 1, wherein fampridine has a d (0.9) particle size between 120 µm and 20 µm or between 100 µm and 25 µm or between 85 µm and 30 µm or between 80 µm and 35 µm.

6. The prolonged-release tablet according to claim 1 or 5, wherein the amount of fampridine is between 0.1% to 5.0% by weight of the total composition.

7. The prolonged-release tablet according to claim 1 or 5, wherein the amount of fampridine is between 1.2% to 3.5% by weight of the total composition.

8. The prolonged-release tablet according to any preceding claims, wherein further comprising at least one pharmaceutically acceptable excipient which is selected from fillers, lubricants, glidants, coating agents or mixtures thereof.

9. The prolonged-release tablet according to claim 8, wherein fillers are selected from the group comprising microcrystalline cellulose, mannitol, spray-dried mannitol, lactose, lactose monohydrate, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or mixtures thereof.

10. The prolonged-release tablet according to claim 9, wherein the filler is microcrystalline cellulose.

11. The prolonged-release tablet according to claim 10, wherein the amount of microcrystalline cellulose is between 40.0% to 65.0% by weight of the total composition.

12. The prolonged-release tablet according to claim 8, wherein lubricants are selected from the group comprising from magnesium stearate, calcium stearate, zinc stearate, talc, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyseryl palmito sulphate, sodium stearyl fumarate, sodium lauryl sulphate or mixtures thereof.

13. The prolonged-release tablet according to claim 8, wherein comprising;
- Fampridine having d (0.9) particle size smaller than 120 µm
- HPMC K100LV
- Microcrystalline cellulose PH103
- Anhydrous colloidal silicon
- Magnesium stearate.
